# EUROPÄISCHE PATENTSCHRIFT

(11) **EP 1 480 630 B1**
(45) Veröffentlichungstag und Bekanntmachung des Hinweises auf die Patenterteilung: **22.04.2009**
(21) Anmeldenummer: 03742953.7
(22) Anmeldetag: 24.02.2003
(51) Int. Cl.: A61K 31/375, A61K 31/4415, A61K 31/519, A61K 31/525, A61K 31/714, A61K 45/06

(54) **VERWENDUNG VON FOLATEN ZUR HERSTELLUNG EINER ZUBEREITUNG GEEIGNET ZUR VORBEUGUNG UND BEHANDLUNG VON ENTZ NDUNGEN UND ENTZ&Uum l;NDUNGSASSOZIIERTER KRANKHEITEN, IM SPEZIELLEN ZUR BEEINFLUSSUNG DER INFLAMMATIONSMARKER CRP UND SAA**
USE OF FOLATES FOR PRODUCING A PREPARATION SUITABLE FOR PREVENTING AND TREATING INFLAMMATION AND DISEASES ASSOCIATED WITH INFLAMMATION, ESPECIALLY FOR INFLUENCING THE INFLAMMATION MARKERS CRP AND SAA
UTILISATION DE FOLATES POUR PRODUIRE UNE PREPARATION CONVENANT A LA PREVENTION ET AU TRAITEMENT DES INFLAMMATIONS ET DES MALADIES ASSOCIEES AUX INFLAMMATIONS, EN PARTICULIER POUR AGIR SUR LES MARQUEURS D'INFLAMMATION CRP ET SAA

(30) Priorität: 26.02.2002 CH 337022002
(43) Veröffentlichungstag der Anmeldung: 01.12.2004
(73) Patentinhaber: Merck Eprova AG, 8200 Schaffhausen (CH)
(72) Erfinder: MUELLER, Thomas, F., Boston, MA 02120 (US); MOSER, Rudolf, CH-8200 Schaffhausen (CH); ULMANN, Martin, CH-8447 Dachsen (CH)
(86) Internationale Anmeldenummer: PCT/EP2003/001848
(87) Internationale Veröffentlichungsnummer: WO 2003/072096

(56) Entgegenhaltungen:
- WO-A-01/85178
- WO-A-01/91734

## Beschreibung

Diese Erfindung bezieht sich auf die Verwendung von Folaten als aktive Wirksubstanz zur Herstellung einer Zubereitung geeignet zur Vorbeugung und Behandlung von Entzündungen, im Speziellen zur Beeinflussung des Spiegels der Entzündungsmarker C-reaktives Protein (CRP) und Serum Amyloid A Protein (SAA). Anwendungsgebiete sind alle Anomalien des CRP- und SAA-Spiegels.

Im vorllegenden Text bezieht sich der Begriff Folate sowohl auf Pteroinsäure-Monoglutamat (Folsäure), wie auch auf reduzierte Formen wie Dihydrofolate und Tetrahydrofolate wie z.B. 5-Formyltetrahydrofolsäure, 5-Methyltetra-hydrofolsäure, 5,10-Methylentetrahydrofolsäure, 5,10-Methenyltetrahydrofolsäure, 10-Formyltetrahydrofolsäure und Tetrahydrofolsäure, deren Polyglutamate, deren optische Isomeren, im Speziellen deren optische reine natürliche Isomeren, aber auch Mlschungen von optischen Isomeren, insbesondere racemische Mischungen, sowie auch deren pharmazeutisch verträgliche Salze.

Folate sind wichtige Cofaktoren in C1-Übertragungsreaktionen und sind beteiligt in Schlüsselsynthesen in menschlichen, tierischen und pflanzlichen Zellen, im Speziellen in der DNA-Biosynthese und im Methylierungskreislauf. Als Arzneimittel wurden Folate bisher vorwiegend als Calcium-Salz der 5-Formyl-5,6,7,8-tetrahydrofolsäure (Leucovorin) oder der 5-Methyl-5,6,7,8-tetrahydrofolsäure (Metafolin) verwendet zur Behandlung von megaloblastischer Folsäure-Anämie, als Antidot zur Verstärkung der Verträglichkeit von Folsäure-Antagonisten, speziell von Aminopterin und Methotrexat in der Krebstherapie ("Antifolate rescue"), zur Verstärkung des therapeutischen Effektes von fluorierten Pyrimidinen und der Behandlung von Autoimmunkrankheiten wie Psoriasis, zur Verstärkung der Verträglichkeit von bestimmten Antiparasitika, etwa Trimethoprim-Sulfamethoxazol sowie zur Verminderung der Toxizität von Dideazatetrahydrofolaten in der Chemotherapie.

Entzündungen induzieren über pro-inflammatorische Cytokine die Synthese von sogenannten Akute-Phase Proteinen. Entgegen des Namens tritt eine Akute-Phase Antwort allerdings nicht nur bei akuten, sondern auch bei chronisch entzündlichen Prozessen auf. Deutlich erhöhte zirkulierende Akute-Phase Parameter findet man bei Infektionen, Traumata, Infarkten, Arthritiden. Abstossungsreaktionen von Organtransplantaten und auch bei Neoplasmen. Des weiteren können kardio- und cerebrovaskuläre Erkrankungen wie auch Adipositas, Diabetes mellitus, Urämie, Hypertonie, starke körperliche Belastung, Substitution von Hormonen, Schlafstörungen, Alkoholabusus, Morbus Alzheimer oder Depressionen, Autoimmunerkrankungen sowie immunologische Krankheitsbilder mit einer erhöhten Akute-Phase Antwort einhergehen. Neben den zugrundeliegenden Erkrankungen können zusätzlich auch die therapeutischen Massnahmen mit einer Entzündungsantwort einhergehen, wie z.B. Hämodialyseverfahren, Lipidapheresebehandlungen, Kathetherdiletationen oder Bestrahlungstherapien [Kushner I, Cleveland Clin J Med 2001; 68 (6): 535-37; Malle et al, Eur J Clin Invest 1996; 26: 427-35; Ridker et al, N Engl J Med 2000; 342: 836-43; Greaves et al, Trends in Immunology 2002; 23 (11): 535-41; Wick et al, Trends in Immunology 2001; 22 (12): 665-9]. Die Höhe der Entzündungsmarker spiegelt nicht nur das Vorliegen, sondern auch die Schwere der Entzündungsreaktion wider, hat prognostische Bedeutung und zeigt im Verlauf das Ansprechen auf die Therapie an. Optimierte Testverfahren haben in den letzten Jahren die diagnostische Wertigkeit von Akute-Phase Markern zur Erfassung gerade der chronisch inflammatorischen Last in den Vordergrund gerückt [Ridker P, Circulation 2001; 103 (13): 1813-18; Patel et al, Cleveland Clin J Med 2001; 68 (6): 521-34]. Insbesondere die Arteriosklerose wird zunehmend als inflammatorische Erkrankung aufgefasst und erhöhte Entzündungsmarker sind wesentliche Risikofaktoren für cardio- und cerebrovaskuläre Ereignisse [Ross R, N Engl J Med 1999; 340: 115-25, Ridker et al, N Engl J Med 1997; 336: 973-9, Haverkate et al, for the European Concerted Action on Thrombosis and Disabilities Angina Pectoris Study Group, The Lancet 1997; 349:462-6; Ridker et al, N Engl J Med 2002; 347 (20): 1557-65].

C-reaktives Protein (CRP) ist ein Eiweiss, das in der Leber gebildet wird und durch seinen schnellen (innerhalb von 12 Stunden) und extrem hohen (bis zu 2'000-fach) Anstieg zu den klassischen Akute-Phase Proteinen gehört [Malle et al, Eur J Clin Invest 1996; 26: 427-35]. Funktionell besitzt es sowohl pro- als auch anti-inflammatorische Eigenschaften. Es bindet eingedrungene Fremdstoffe, aktiviert Makrophagen und das Komplementsystem, induziert Cytokinfreisetzung und reguliert Leukozytenakkumulation und Adhesion [Patel et al, Cleveland Clin J Med 2001; 68: 521-34; Greaves et al, Trends in Immunology 2002; 23 (11): 535-41]. Zusätzlich zeigen aktuelle Untersuchungen, dass CRP auch direkt pro-inflammatorisch auf humane Endothelzellen wirkt [Pasceri et al, Circulation 2000; 102: 2165-8]. Primär ist es der angeborenen, unspezifischen Immunantwort zuzuordnen. Der Referenz-/Normalwert von CRP im Plasma liegt bei Werten bis 2 mg/l (Erwachsene und Kinder), wobei je nach verwendetem Test und Untersuchungsgruppe unterschiedliche Normalbereiche angegeben werden.

Da die Halbwertszeit mit 24 Stunden relativ kurz ist, machen sich Veränderungen im entzündlichen Geschehen direkt in der CRP-Konzentration bemerkbar. CRP steigt bei bakteriellen Entzündungen am schnellsten (innerhalb weniger Stunden) und am stärksten an. Bei viralen oder lokalen Infektionen und chronischen Entzündungen erfolgt ein geringerer Anstieg des CRP. Aufgrund der sehr sensitiven modernen Assays für CRP (high sensitive CRP) eignet sich CRP sehr gut für Verlaufsbeobachtungen von entzündlichen Erkrankungen [Roberts et al, Clin Chem 2001; 47: 418-25]. Bei erfolgreicher Antibiotika-Therapie fällt es schnell wieder ab und zeigt intra-individuell bei fehlender Entzündungsaktivität eine sehr geringe Variabilität sowohl diurnal als auch im Langzeitverlauf [Ockene et al, Clin Chem 2001; 47: 444-50]. Schon geringe Erhöhungen von CRP ohne klinische Zeichen einer Entzündung korrelieren mit einer deutlich erhöhten kardio- und cerebrovaskulären Morbidität und Mortalität [Ridker et al, N Engl J Med 1997; 336: 973-9; Haverkate et al. for the European Concerted Action on Thrombosis and Disabilities Angina Pectoris Study Group, The Lancet 1997; 349: 462-6; Harris et al, Am J Med 1999; 106: 506-12; Ridker et al, N Engl J Med 2002; 347 (20): 1557-65]. Aspirin scheint in diesem Zusammenhang seine günstige Wirkung auch durch die anti-inflammatorische Aktivität zu entfalten und ein leicht erhöhtes CRP ist ein wichtiger Marker für die Notwendigkeit dieser Therapie [Ridker et al, N Engl J Med 1997; 336: 973-9]. Inwieweit CRP dabei nur Surrogatmarker oder auch etiologisch wichtiger Faktor ist, ist noch unklar [Graeves et al, Trends in Immunology 2002; 23 (11): 535-41]. Der kürzlich beschriebene CRP-senkende Effekt von Statinen unterstützt die Schlüsselrolle der Inflammation bei der Arteriosklerose und die Senkung selbst minimal erhöhter CRP-Spiegel ist nach diesen aktuellen Studien vergleichbar wichtig wie die Cholesterinsenkung [Albert et al, for the PRINCE Investigators, JAMA 2001; 286 (1): 64-70; Ridker et al, N Engl J Med 2001; 344 (26): 1959-65].
Erhöhungen auf 10-100 mg/l zeigen leicht bis mässig, in der Regel akute entzündllche Prozesse oder solche geringer Ausdehnung an. Dazu gehören die lokale bakterielle Infektion, unkomplizierte Zystitis, Bronchitis. Traumen, postoperative Entzündungsreaktion. Unfall, Myokardinfarkt, Tuberkulose oder Sarkoidose. Werte von 100 mg/l und mehr bei akuten Krankheitsgeschehen sprechen für hohe und/oder ausgedehnte Entzündungsaktivität. Dazu zählen Sepsis, größere Traumen, bakterielle Infektionen, metastasierende Tumoren, aktive rheumatoide Arthritis, seronegative Spondylarthritis, Immunvaskulitis, Polymyalgia rheumatica, Morbus Crohn oder tiefe Venenthrombose.

Serum amyloid A (SAA) Protein besteht aus einer Familie von polymorphen Apolipoproteinen, die hauptsächlich in der Leber synthetisiert werden. SAA ist ein sehr empfindlicher Marker der Akute-Phase Antwort und reagiert bei Entzündung, Nekrose, Abstossungsreaktion und auch Tumor-Aussaat. SAA ist ein α1-Globulin bestehend aus einer einfachen Polypeptidkette mit einem Molekulargewicht zwischen 11'500 und 14'000 Dalton und zirkuliert im Blut an HDL gebunden [Malle et al, Eur J Clin Invest 1996; 26: 427-35].

Der Referenz-/Normalwert von SAA im Plasma liegt bei Werten bis 1 mg/l. Bei Entzündungen steigt die SAA-Konzentration innerhalb von wenigen Stunden auf Werte bis 2000 mg/l. In der Regel verlaufen CRP- und SAA-Werte parallel, allerdings scheint SAA etwas früher und dynamischer zu reagieren und auch höher anzusteigen als CRP [Gabay et al, N Engl J Med 1999; 340: 448-54; Liuzzo et al, N Engl J Med 1994; 331: 417-24; Wilkins et al, Clin Chem 1994; 40: 1284-90; Malle et al, Eur J Clin Invest 1996; 26: 427-35].

Erhöhte CRP- und SAA-Werte stehen im Zusammenhang mit einer Reihe von Erkrankungen, im Speziellen mit Entzündungen und entzündungsassoziierter Krankheiten, wie z.B. akut entzündliche, nekrotisierende und tumorartige Erkrankungen, akute Gewebsläsionen, bakterielle und virale Infektionen, rheumatische Erkrankungen wie rheumatoide Arthritis, Polyarthritis, Spondylarthrltis ankylopoetica, Meningitis, Lungenentzündung, Pyelonephritis, akute Bronchitis. Tuberkulose, Sepsis und akute Pankreatitis, Morbus Alzheimer, postoperative Komplikationen, rheumatische Erkrankungen, maligne Tumoren, Abstossungsreaktionen, akute Herzinfarkte, Reiter- Syndrom, Arthropathia psoriatica, Colitis ulcerosa, Morbus Crohn, usw. Des weiteren können kardio- und cerebrovaskuläre Erkrankungen wie auch Adipositas, Diabetes mellitus, Urämie, Hypertonie, starke körperliche Belastung, Substitution von Hormonen, Schlafstörungen, Alkoholabusus, Morbus Alzheimer, Anämie oder Depressionen, Organtransplantationen, Auto-Immunerkrankungen und immunologische Krankheiten mit einer erhöhten Akute-Phase Antwort und entsprechend erhöhten SAA und CRP Werten einhergehen. Ausserdem kann es zu leichten, noch im Bereich des Normalwerts, aber trotzdem mit einem erhöhten Risiko für Komplikationen einhergehenden, Anstiegen der genannten Entzündungsmarker kommen, die zukünftig schon als Indikation zur Therapie angesehen werden können. Dazu gehört auch der mit dem Begriff "inflamm-aging" bezeichnete Prozess der parallel zum Altern zunehmenden Entzündungslast [Kushner I, Cleveland Clin J Med 2001; 68 (6): 535-37; Malle et al, Eur J Clin Invest 1996; 26: 427-35; Ridker et al, N Engl J Med 2000; 342: 836-43; Neumann et al, Pteridines 1998; 9: 113-21; Müller TF, Papst Science Publ., Lengerich 1999, 175 pp].

Die Verwendung von Folaten als aktive Wirksubstanz zur Herstellung einer Zubereitung geeignet zur Vorbeugung oder zur Behandlung von Entzündungen und entzündungsassoziierter Krankheiten, im Speziellen zur Beeinflussung des Spiegels der lnflammationsmarker CRP und SAA, wurde bisher weder vorgeschlagen noch beschrieben.

Es wurde nun überraschend gefunden, dass die Verwendung von Zubereitungen enthaltend Folate als aktive Wirksubstanz geeignet ist zur Behandlung und zur Vorbeugung von Entzündungen, im Speziellen zur Beeinflussung des Spiegels der Inflammationsmarker CRP und SAA.

Als Folate eingesetzt werden können sowohl Pteroinsäure-Monoglutamat (Folsäure), wie auch reduzierte Formen wie Dihydrofolate und Tetrahydrofolate, deren Polyglutamate, deren optische Isomeren und deren pharmazeutisch verträgliche Salze. Bevorzugt eingesetzt als Folate sind Tetrahydrofolate, im Speziellen die natürlichen stereoisomeren Formen von Tetrahydrofolaten wie 5-Formyl-(6S)-tetrahydrofolsäure, 5-Methyl-(6S)-tetrahydrofolsäure, 5,10-Methylen-(6R)-tetrahydrofolsäure, 5,10-Methenyl-(6R)-tetrahydrofolsäure, 10-Formyl-(6R)-tetrahydrofolsäure, 5-Formimirlo-(6S)-tetrahydrofolsäure oder (6S)-Tetrahydrofolsäure oder deren pharmazeutisch verträglichen Salze. Die verwendeten Folate können im Folatstoffwechsel in der Regel gegenseitig ineinander umgewandelt werden. Bevorzugt werden aber 5-Methyl-(6S)-tetrahydrofolsäure, 5-Formyl-(6S)-tetrahydrofolsäure und deren pharmazeutisch verträglichen Salze verwendet.

Pharmazeutisch verträgliche Salze sollten sowohl pharmakologisch wie auch pharmazeutisch verträglich sein. Solche pharmakologisch und pharmazeutisch verträgliche Salze können Alkali- oder Erdalkalimetall-Salze sein, vorzugsweise Natrium-, Kalium-, Magnesium- oder Calcium-Salze.

Zubereitungen beziehen sich auf enterale (z. B. oral, sublingual oder rektal), parenterale oder topische (z. B. transdermale) Formen. Als Träger können organische oder anorganische Substanzen verwendet werden, die nicht mit der aktiven Wirksubstanz reagieren, z. B. Wasser, Oel, Benzylalkohol, Polyethylenglycol, Glycerintriacetat oder andere Fettsäureglyceride, Gelatine, Lecithin, Cyclodextrine, Kohlenhydrate wie Lactobiose oder Stärke, Magnesiumstearat, Talk oder Cellulose. Bevorzugt bei der oralen Anwendung sind Tabletten, Dragées, Kapseln, Pulver, Sirup, Konzentrate oder Tropfen, für die rektale Anwendung bevorzugt sind Suppositorien, zur parenteralen Anwendung bevorzugt eingesetzt werden Lösungen, wasser- oder oelbasierend oder Lyophilisate.
Ebenfalls anwendbar sind Suspensionen, Emulsionen oder Implantate und für die topische Anwendung Pflaster oder Cremes. Zubereitungen für die parenterale Anwendung beinhalten sterile wässrige und nichtwässrige Injektionslösungen der aktiven Verbindungen, die vorzugsweise isotonisch zum Blut des Empfängers sind.

Diese Zubereitungen können Stabilisatoren, Additive für die kontrollierte Freisetzung der pharmazeutisch aktiven Verbindungen, Antioxidantien, Puffer, Bakteriostatikas und Hilfsstoffe zur Einstellung einer isotonischen Lösung beinhalten. Wässrige und nichtwässrige sterile Suspensionen können Suspensionszusatzstoffe und Verdickungsmittel beinhalten. Die Zubereitung kann als Einfachdosis- oder als Mehrfachdosis-Behälter vorhanden sein, zum Beispiel als verschweisste Ampullen und kann als gefriergetrocknetes (lyophilisiertes) Produkt gelagert und bei Bedarf mit steriler Flüssigkeit, zum Beispiel Wasser oder Salzlösung, für den Gebrauch vorbereitet werden. In derselben Art und Weise können auch steriles Pulver, Granulat oder Tabletten verwendet werden. Alle Zubereitungen können zusätzlich eine oder mehrere separat oder synergistisch wirkende aktive Verbindungen enthalten. Im Speziellen sind dies Stoffe, die im Folatzyklus eine Rolle spielen, beziehungsweise den Folatzyklus beeinflussen oder eine zusätzliche antientzündliche Wirkung haben wie Vitamine, Antioxidantien wie Vitamin E oder Betacarotin, Radikalfänger, Biopterine und/oder andere Wirkstoffe. Beispiele sind Vitamin B₂, B₆, B₁₂ oder Vitamin C, Glutathion, Acetylcystein, Betain, Biopterine in allen Oxidationsstufen und isomeren Formen wie Biopterin, Im Speziellen L-erythro-Biopterin, 7,8-Dihydrobiopterin. 5,6,7,8-Tetrahydrobiopterin, im Speziellen L-Sepiapterin, D-Neopterin, Xanthopterin, 6-Hydroxymethylpterin. Zusätzlich zählen dazu die Lipidsenker wie Clofibrinsäurederivate (Fibrate), z.B. Clofibrat, Bezafibrat, Etofibrat, Fenofibrat), lonenaustauscherharze, z.B. Colestyramine oder Colestipol, Nikotinsäure (-derivate), z.B. Acipimox, Sitosterin und HMG-CoA-Reduktasehemmer, z.B. Atorvastatin, Lovastatin, Pravastatin, Simvastatin, Fluvastatin oder Cerivastatin. Zu den weiteren Substanzgruppen gehören Immunosuppressiva wie Kortikosteroide, Mycophenolat, Mofetil, Rapamycin, Calcineurininhibitoren, mono- und polyklonale Antikörper; Wachstumsfaktoren wie Erythropoetin oder GM-CSF. Als weitere Substanzgruppe gehören zu diesen Stoffen nichtsteroidale anti-inflammatorische Substanzen wie Pentoxyfyllin, Sulfasalazin, Gold, Aspirin, Omega-3 Fettsäuren, Thrombozytenaggregationshemmer wie Glykoprotein IIb/IIIa Rezeptor Inhibitoren, Hormone, Flavinoide oder weitere nichtsteroidale anti-inflammatorische Carboxylsäuren wie Aspirin, Salsalate, Diflunisal oder Cholin Magnesium Trisalicylsäure, oder weitere nichtsteroidale anti-inflammatorische Proprionsäuren wie Ibuprofen, Naproxen, Fenoprofen, Ketoprofen, Flurbiprofen oder Oxaprozin, oder weitere nichtsteroidale anti-inflammatorische Essigsäurederivate wie Indomethacin, Tolmetin, Sulindac, Diclofenac oder Etodolac, oder weltere nichtsteroidale anti-inflammatorische Fenamate wie Meclofenamat oder Mefenamic acid, oder weitere nichtsteroidale anti-inflammatorische Enolsäurederivate wie Piroxicam oder Phenylbutazone, oder weitere nichtsteroidale anti-inflammatorische Naphthylkanones wie Nabumetone, sowie COX-2 Inhibitoren wie Celecoxib oder Rofecoxib. Ausserdem gehören dazu Substanzen mit antiinflammatorischer Wirkung wie Betablocker, Antikörper gegen Cytokine, z.B. anti-TNFalpha Antikörper, oder Perfusionslösungen zur Organkonservierung wie Eurocollins, HTK oder University of Wisconsin (UW) Lösung.

Die Zubereitung beinhaltet zwischen 0.001 mg und 1'000 mg der aktiven Wirksubstanz pro Dosis. In der Prophylaxe werden Zubereitungen enthaltend vorzugsweise zwischen 5 µg und 1'000 µg der aktiven Wirksubstanz pro Dosis eingesetzt. In der Therapie werden Zubereitungen enthaltend vorzugsweise zwischen 0.1 mg und 200 mg der aktiven Wirksubstanz pro Dosis eingesetzt. Die Dosierung hängt ab von der Therapieform, von der Anwendungsform der Zubereitung, vom Alter, Gewicht, der Ernährung und Zustand des Patienten. Die Behandlung kann mit tiefer Dosierung unterhalb der optimalen Menge begonnen und bis zur Erreichung des optimalen Effektes gesteigert werden. Bevorzugt kann sich die Dosierungen in der Prophylaxe zwischen 5 µg und 5'000 µg pro Tag, im Speziellen zwischen 100 µg und 1'000 µg pro Tag bewegen. Optimale Dosierungen in der Therapie bewegen sich zwischen 0.1 mg und 100 mg pro Tag, im Speziellen zwischen 0.5 mg und 5 mg pro Tag. Die Anwendung kann entweder als Einmal-Gabe oder als wiederholte Dosierung erfolgen.

Die Zubereitungen können zur Vorbeugung und Behandlung von Entzündungen und entzündungsassoziierter Krankheiten beim Menschen wie auch beim Tier eingesetzt werden.

Die folgenden Beispiele können mit ähnlichem Erfolg durchgeführt werden durch Ersetzen der generisch oder spezifisch beschriebenen Produkte und/oder Verfahrensbedingungen dieser Erfindung durch solche, die in den folgenden Beispielen aufgeführt sind. Ebenso sind die folgenden spezifischen Ausführungsformen rein beispielhaft und in keiner Art und Weise limitierend auf den Rest der Offenbarung zu sehen.

### Beispiele zur Illustrierung der Erfindung

### Beispiel 1

### Tablette enthaltend 1 mg 5-Formyl-(6S)-tetrahydrofolsäure

Eine Mischung von 13.3g 5-Formyl-(6S)-tetrahydrofolsäure-Calciumsalz-Pentahydrat (entsprechend 10g 5-Formyl-(6S)-tetrahydrofolsäure), 4 kg Lactose, 1.2 kg Stärke, 0.2 kg Talk und 0.1 kg Magnesiumstearat werden zu Tabletten gepresst, so dass jede Tablette 1 mg 5-Formyl-(6S)-tetrahydrofolsäure enthält.

Die Tablette kann beschichtet als Filmtablette oder gemahlen und in Kapseln abgefüllt verwendet werden.

### Beispiel 2

### Suppositorien enthaltend 60 mg 5-Mothyl-(6S)-tetrahydrofolsäure

Eine Mischung von 632 g 5-Methyl-(6S)-tetrahydrofolsäure-Calciumsalz-Pentahydrat (entsprechend 500 g 5-Methyl-(6S)-tetrahydrofolsäure), 50 g Hydroxypropylcellulose und 2 kg semisynthetische Glyceride werden zu Suppositorien geschmolzen, so dass jedes Suppositorium 500 mg 5-Methyl-(6S)-tetrahydrofolsäure enthält.

### Beispiel 3

### Injektionslösung enthaltend 0.5 mg 5-Mothyl-(6S)-tetrahydrofolsäure

0.5 g 5-Methyl-(6S)-tetrahydrofolsäure, 10 g Glutathion, 30 g Zitronensäure, 160 g Mannitol, 1 g Methyl-p-hydroxybenzoesäure, 17.7 g Natriumhydroxid (oder die notwendige Menge um einen pH-Wert der Lösung von 7,3 bis 7.8 einzustellen) werden auf 3 Liter Wasser zur Injektion gelöst und in Ampullen abgefüllt, so dass jede Ampulle 0.5 mg 5-Methyl-(6S)-tetrahydrofolsäure enthält.

### Beispiel 4

### Injizierbares Lyophillsat enthaltend 1 mg (6S)-Tetrahydrofolsäure

Eine Lösung von 1 g (6S)-Tetrahydrofolsäure-Natriumsalz in 1'000 ml doppelt destilliertem Wasser wird in Ampullen sterilfiltriert und lyophilisiert, so dass jede Ampulle 1 mg (6S)-Tetrahydrofolsäure enthält.

Tetrahydrofolsäure ist sehr sauerstoffempfindlich, daher muss strikte sauerstoff-frei gearbeitet werden. Der Einsatz eines Oxidationsschutzmittels wie Ascorbinsäure kann notwendig sein.

### Beispiel 5

### Injizierbares Lyophilisat enthaltend 20 mg 5,10-Methylen-(6R)-tetrahydrofolsäure

Eine Lösung von 10 g β-Hydroxypropyl-Cyclodextrin-Eirischlussverbindung von 5,10-Methylen-(6R)-tetrahydrofolsäure-Natriumsalz in 2'000 ml doppelt destilliertem Wasser wird in Ampullen sterilfiltriert, so dass jede Ampulle 20 mg 5,10-Methylen-(6R)-tetrahydrofolsäure enthält.

Für 5,10-Methylentetrahydrofolsäure gelten die gleichen Vorsichtsmassnahmen wie für Tetrahydrofolsäure (Beispiel 4).

### Beispiel 6

### Tablette enthaltend 0.4 mg 5-Formyl-(65)-tetrahydrofolsäure

Eine Mischung von 5.32 g 5-Formyl-(6S)-tetrahydrofolsäure-Calciumsalz-Pentahydrat (entsprechend 4 g 5-Formyl-(6S)-tetrahydrofolsäure), 4 kg Lactose, 1.2 kg Stärke, 0.2 kg Talk und 0.1 kg Magnesiumstearat werden zu Tabletten gepresst, so dass jede Tablette 4 mg 5-Formyl-(6S)-tetrahydrofolsäure enthält.

Die Tablette kann beschichtet als Filmtablette oder gemahlen und in Kapseln abgefüllt verwendet werden.

### Beispiel 7

### Injizierbares Lyophillsat enthaltend 100 µg S-Methyl-(6S)-tetrahydrofolsäure

Eine Lösung von 100 mg 5-Methyl-(6S)-tetrahydrofolsäure-Natrlumsalz in 1'000 ml doppelt destilliertem Wasser wird unter Schutzgas in Ampullen sterilfiltriert und lyophilisiert, so dass jede Ampulle 100 µg 5-Methyl-(6S)-tetrahydrofolsäure enthält.

Tetrahydrofolsäure ist sehr sauerstoffempfindlich, daher muss strikte sauerStoff-frei gearbeitet werden. Der Einsatz eines Oxidationsschutzmittels wie Ascorbinsäure kann notwendig sein.

### Beispiel 8

### Tablette enthaltend 15 mg 5-Methyl-(6S)-tetrahydrofolsäure

Eine Mischung von 19.18g 5-Methyl-(6S)-tetrahydrofolsäure-Calciumsalz-Pentahydrat (entsprechend 15 g 5-Methyl-(6S)-tetrahydrofolsäure), 120g Lactose, 21.5 g Maisstärke, 7.08g Acetylcellulose, 2.28 g Diethylphthalat, 0.64 g Silicone HK-15 und 2 g Magnesiumstearat werden zu Tabletten gepresst, so dass jede Tablette 15 mg 5-Methyl-(6S)-tetrahydrofolsäure enthält.

Die Tablette kann beschichtet als Filmtablette oder gemahlen und in Kapseln abgefüllt verwendet werden.

### Beispiel 9

### Tabletten enthaltend 15 mg 5-Methyl-(6R,S)-tetrahydrofolsäure

In analoger Weise, wie im Beispiel 8 beschrieben, werden Tabletten enthaltend 15 mg 5-Methyl-(6R,S)-tetrahydrofolsäure mit Maisstärke, Milchzucker, Magnesiumstearat, Polyethylenglycol 6000, Polymetacrylat, Polysorbit 80, Dimethylpolysiloxan, Natriumhydroxid und Talk hergestellt.

### Beispiel 10

### Tabletten enthaltend 15 mg 5-Formyl-(6R,S)-tetrahydrofolsäure

In analoger Weise, wie im Beispiel 8 beschrieben, werden Tabletten enthaltend 15 mg 5-Formyl-(6R,S)-tetrahydrofolsäure mit Maisstärke, Milchzucker, Magnesiumstearat, Polyethylenglycol 6000, Polymetacrylat, Polysorbit 80, Dimethylpolysiloxan, Natriumhydroxid und Talk hergestellt.

### Beispiel 11

### Kombinationspräparat aus 5-Mothyl-(6S)-tetrahydrofolsäure, Vitamin B₆ und Vitamin B₁₂

Für Präparate zur oralen Anwendung wird eine Filmtablette formuliert, die folgende Bestandteile enthält;

| | |
|---|---|
| 10 mg | 5-Methyl-(6S)-tetrahydrofolsäure |
| 100 mg | Vitamin B₆ |
| 1 mg | Vitamin B₁₂ |
| | pharmazeutisch verträgliche Hilfsstoffe |

Das Kombinationspräparat kann auch als Lösung z. B. für die parenterale Anwendung formuliert werden.

### Beispiel 12

### Basisvitaminpräparat enthaltend u.a. 5-Methyl-(6S)-tetrahydrofolsäure

Für Präparate zur oralen Anwendung wird eine Filmtablette formuliert, die folgende Bestandteile enthält:

| | |
|---|---|
| 0.4 mg | 5-Methyl-(6S)-tetrahydrofolsäure |
| 3 mg | Vitamin B₁ |
| 1.7 mg | Vitamin B₂ |
| 10 mg | Vitamin B₆ |
| 0.006 mg | Vitamin B₁₂ |
| 60 mg | Vitamin C |
| 0.3 mg | Biotin |
| 20 mg | Nicotinamid |
| 10 mg | Pantothensäure |
| | pharmazeutisch verträgliche Hilfsstoffe |

Das Kombinationspräparat kann auch als Lösung z. B. für die parenterale Anwendung formuliert werden.

### Beispiel 13

### Kombinationspräparat enthaltend u.a. 5-Methyl-(6S)-tetrahydrofolsäure und Betain

Analog zu den Beispielen 11 und 12 wird ein Kombinationspräparat hergestellt, enthaltend zusätzlich zur für die entsprechende Anwendung üblichen Menge an 5-Methyl-(6S)-tetrahydrofolsäure auch die für diese Anwendung übliche Menge an Betain.

### Beispiel 14

### Kombinationspräparat enthaltend u.a. 5-Methyl-(6S)-tetrahydrofolsäure und Tetrahydrobiopterin

Analog zu den Beispielen 11 und 12 wird ein Kombinationspräparat hergestellt, enthaltend zusätzlich zur für die entsprechende Anwendung üblichen Menge an 5-Methyl-(6S)-tetrahydrofolsäure auch die für diese Anwendung übliche Menge an Tetrahydrobiopterin.

### Beispiel 15

### Kombinationspräparat enthaltend u.a. 5-Methyl-(6S)-tetrahydrofolsäure und Statine

Analog zu den Beispielen 11 und 12 wird ein Kombinationspräparat hergestellt, enthaltend zusätzlich zur für die entsprechende Anwendung üblichen Menge an 5-Methyl-(6S)-tetrahydrofolsäure auch die für diese Anwendung übliche Menge an Statinen wie z.B. Atorvastatin, Lovastatin, Pravastatin, Simvastatin, Fluvastatin oder Cerivastatin.

### Beispiel 16

### Kombinationspräparat enthaltend u.a. 5-Methyl-(6S)-tetrahydrofolsäure und Aspirin

Analog zu den Beispielen 11 und 12 wird ein Kombinationspräparat hergestellt, enthaltend zusätzlich zur für die entsprechende Anwendung üblichen Menge an 5-Methyl-(6S)-tetrahydrofolsäure auch die für diese Anwendung übliche Menge an Aspirin.

### Beispiel 17

### Kombinationspräparat enthaltend u.a. 5-Methyl-(6S)-tetrahydrofolsäure und zusätzliche Wirkstoffe

Analog zu den Beispielen 11 und 12 wird ein Kombinationspräparat hergestellt, enthaltend zusätzlich zur für die entsprechende Anwendung üblichen Menge an 5-Methyl-(6S)-tetrahydrofolsäure auch die für diese Anwendung übliche Menge an Vitamin B₂, Vitamin B₆, Vitamin B₁₂ oder Vitamin C, Gluthation, Acetylcystein, Pentoxifyllin, Omega-3 Fettsäuren, Vitamin E, Thrombozytenaggregationshemmer wie Glykoprotein Ilb/Illa Rezeptor Inhibitoren, Betablocker, Hormone, Flavinoide oder weitere nichtsteroidale anti-inflammatorische Substanzen wie Ibuprofen, Indomethacin, Diclofenac, Piroxicam, COX-2 Inhibitoren oder Immunsuppressiva, Perfusionslösungen oder anti-inflammatorisch wirksame Antikörper.

### Ermittlung klinischer Daten

Klinische Daten wurden über die im Folgenden beschriebene, prospektive, randomisierte Doppelblind-Studie erhalten.

141 Patientinnen mit terminaler Niereninsuffizienz und einer Behandlung mit chronischer Hämodialyse an 3 Tagen pro Woche im Alter zwischen 24 und 90 Jahren wurden anhand des Patientlnnengeschlechtes und des Vor- bzw. Nichtvorliegens einer Punktmutation C677T auf dem Gen für die Methylenetetrahydrofolat-Reduktase randomisiert in 4 Therapiegruppen zugeteilt. Die Randomisierung aufgrund der Methylenetetrahydrofolat-Reduktase Mutation sollte verhindern, dass ein Ungleichgewicht innerhalb der einzelnen Therapiegruppen hinsichtlich der Enzymaktivität und somit auch des Folsäuremetabolismus entstand.

Alle Patientinnen erhielten als Basisvitaminsupplementierung täglich eine Filmtablette mit der folgenden Zusammensetzung:

| | |
|---|---|
| Vitamin B₁ | 3 mg |
| Vitamin B₂ | 1,7 mg |
| Vitamin B₆ | 10 mg |
| Vitamin B₁₂ | 6 µg |
| Vitamin C | 60 mg |
| Biotin | 0,3 mg |
| Folsäure | 1 mg |
| Nicotinamid | 20 mg |
| Pantothensäure | 10 mg |

Die Vitamine B₆ (Pyridoxin) und B₁₂ (Cobalamin) spielen als Cofaktoren im Folsäuremetabolismus eine wichtige Rolle. Sie sollten deshalb ebenfalls supplementiert werden, zum einen zur Erhöhung der Wirksamkeit der Folate und zum anderen, um eine neurologische Schädigung durch Vitamin B₁₂ Mangel zu vermeiden [Bostom et al, Kidney Int 1997; 52: 10-20; Homocysteine lowering trialists' collaboration, BMJ 1998; 316: 894-8, Bostom et al, Circulation 2000; 101: 2829-32].
Bei sämtlichen Studienpatientlnnen wurde bereits bei Beginn der Studie durch die oben genannte Basisvitaminsubstitution ein einheitlicher Vitaminstatus erzielt und ausgeprägte Vitaminmangelzustände vermieden.

Wie folgend graphisch dargestellt erhielten die Patientlnnen zusätzlich zur bereits bestehenden Basisvitaminpräparation über insgesamt 6 Wochen
- Gruppe PGA 7.5 mg Folsäure pro Tag
- Gruppe FTHF 15 mg 5-Formyl-(6R,S)-tetrahydrofolsäure pro Tag
- Gruppe MTHF 15 mg 5-Methyl-(6R,S)-tetrahydrofolsäure pro Tag
- Gruppe PLAC Placebo

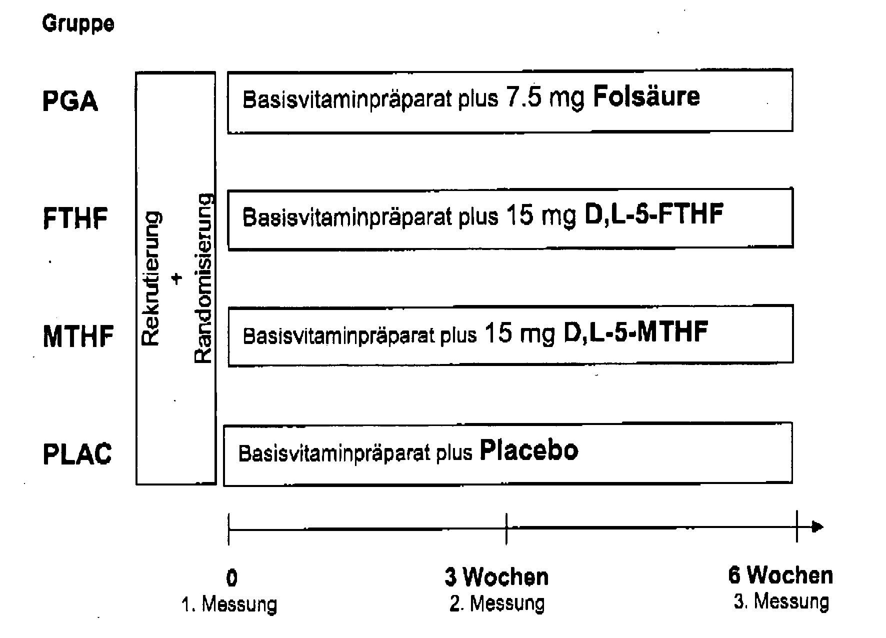

Die erste Blutentnahme im Rahmen der Studie erfolgte vor Beginn der zusätzlichen Vitamingabe. Die zweite Blutprobe wurde 3 Wochen nach Beginn und die dritte zum Abschluss der Studie, d.h. nach 6 Wochen entnommen.

Die folgenden Messgrössen wurden bei den 3 Blutentnahmen jeweils aus Plasmaproben bestimmt:
- Homocystein (totales) (Hcys)
- Gesamtfolate (Fol)
- Vitamin B₁₂ (B12)
- Vitamin B₆ (B6)
- Neopterin (NEOP)
- Kreatinin (Krea)
- LDL- und HDL-Cholesterin (HDL-Chol, LDL-Chol)
- Triglyzeride (TG)
- C-reaktives Protein (CRP)
- Amyloid A Protein (SAA)

Es wurden die Parameter Homocystein, Gesamtfolate sowie Vitamin B₆ und B₁₂ im Plasma der Patientinnen bestimmt. Wegen der bekannten Assoziation zwischen Arteriosklerose und Lipidstoffwechsel erfolgte zusätzlich die Messung der Triglyzeride und Cholesterinfraktionen LDL und HDL. Zusätzlich wurde in den Plasmaproben Neopterin als Parameter einer Immunantwort gemessen. Als Marker für eine inflammatorische Antwort wurde, wie oben ausgeführt, CRP und SAA bestimmt.
Die Blutentnahme erfolgte jeweils zu Beginn der Dialysebehandlung über die liegende Dialysenadel. Pro Blutentnahme wurden für die Untersuchungen insgesamt 30 ml Vollblut abgenommen.

### Ergebnisse der klinischen Studie

Im Folgenden werden die Studienergebnisse in Bezug auf die inflammatorischen Marker CRP und SAA dargestellt.

Die drop-out Rate lag bei 14.9%, d.h. von den insgesamt 141 Patientinnen, die in die Randomisierung eingingen, haben 121 die Studie abgeschlossen. Bei der Auswertung werden sämtliche erhobenen Messwerte berücksichtigt.

Neben deskriptiver Statistik wurden insbesondere die parametrischen Testverfahren ANOVA (Analysis of Variance), Student t-Test, Korrelations- und 'matched pair' Analysen mit dem JMP-SAS Statistikpaket durchgeführt.

### Verteilung der Basischarakteristika

Die folgende Tabelle 1 zeigt eine Übersicht zu den wesentlichen Laborwerten sowie deren Verteilung, Mittelwert und Bereich bzw. Standardabweichung vor Beginn der Folattherapie.

**Tabelle 1**

| **Gruppe** | **Gesamt** | **FTHF** | **MTHF** | **PGA** | **PLAC** | **Prob>F³** |
|---|---|---|---|---|---|---|
| **Zahl [n]** | **141** | **37** | **35** | **36** | **33** | |
| **Frauen [n]** | **57** | **14** | **14** | **14** | **15** | |
| **Alter** [a]¹ | **64** | **60** | **68** | **67** | **60** | |
| | 24-90 | 24-89 | 44-90 | 43-87 | 37-80 | |
| **Hcys** [µmol/l]² | **28.8** | **30.3** | **28.5** | **27.7** | **28.3** | 0.87 |
| | 13.9 | 13.5 | 12.7 | 13.4 | 16.3 | |
| **Folat** [nmol/l]² | **75.2** | **62.3** | **80.9** | **84.6** | **72.6** | 0.53 |
| | 68.5 | 64.8 | 58.4 | 93.2 | 46.7 | |
| **CRP** [mg/l]² | **14.1** | **16.3** | **18.9** | **9.6** | **11.3** | 0.37 |
| | 24.7 | 38.4 | 21.9 | 8.6 | 18.2 | |
| **SAA** [mg/l]² | **23.9** | **28.4** | **34.5** | **11.2** | **21.3** | 0.56 |
| | 71.7 | 103 | 72.7 | 15.5 | 65.2 | |
| **NEOP** [µmol/mol crea]² | **185** | **162** | **174** | **238** | **164** | 0.28 |
| | 185 | 111 | 95 | 326 | 88 | |

| | | | | | | |
|---|---|---|---|---|---|---|
| ¹ Mittelwert und Bereich ² Mittelwert und Standardabweichung ³ ANOVA | | | | | | |

Sowohl in der ANOVA als auch im Student t-Test ergaben sich für keinen der dargestellten Parameter für die Ausgangswerte signifikante Unterschiede zwischen den einzelnen Therapiegruppen. Damit war die Randomisierung erfolgreich.

### Höhe der Akute-Phase Proteine bei den untersuchten Dlalysepatientinnen

CRP und SAA wurden beide mittels Immunnephelometrie und Tests der Firma DadeBehring (N High Sensitivity CRP und N Latex SAA assays) gemessen. Die für die verwendeten Tests ermittelten Referenzwerte für Gesunde sind:
CRP - 1.6 mg/l Mittelwert, 1.1 mg/l Median, 5 mg/l 95% Perzentile
SAA - 2.6 mg/l Mittelwert, 2.0 mg/l Median, 6.8 mg/l 95% Perzentile,
Die untersuchten Dialysepatientlnnen zeigen im Mittel ca. 10-fach erhöhte SAA bzw. CRP-Werte.

### Korrelationen zwischen den einzelnen Parametern

Eine Korrelationsanalyse für die Ausgangswerte von CRP und SAA ergab die in der folgenden Tabelle 2 dargestellten Werte.

**Tabelle 2**

| | Pearson | p-Wert |
|---|---|---|
| SAA vs. CRP | 0.912 | *** |

| | | |
|---|---|---|
| *** i.e. p-Wert <0.001 | | |

Wie dargestellt korrelieren die Akute-Phase Proteine SAA und CRP hochsignifikant. Dagegen ergaben sich keine Korrelationen zwischen den Ausgangswerten von Neopterin und CRP bzw. SAA. Ebenso wenig korrelierten die Homocystein- und Folatwerte vor Therapie mit den Entzündungsmarkern (Daten nicht dargestellt).

### Effekte der Folattherapie auf die Parameter

Die Effekte der Folattherapie werden im Folgenden zunächst als Histogramm dargestellt.
Die Abbildungen 1 und 2 zeigen als Balkenhistogramm die Mittelwerte und Standardabweichungen der untersuchten Parameter für die verschiedenen Therapiegruppen zu Beginn der Studie (BL baseline), nach 3 Wochen (3 w) und 6 Wochen (6 w) Folatgabe. Zusätzlich sind die Medianwerte im Therapieverlauf graphisch in den Abbildungen 3 und 4 dargestellt. Die Messwerte sind zusätzlich tabellarisch in Tabelle 3 dargestellt.

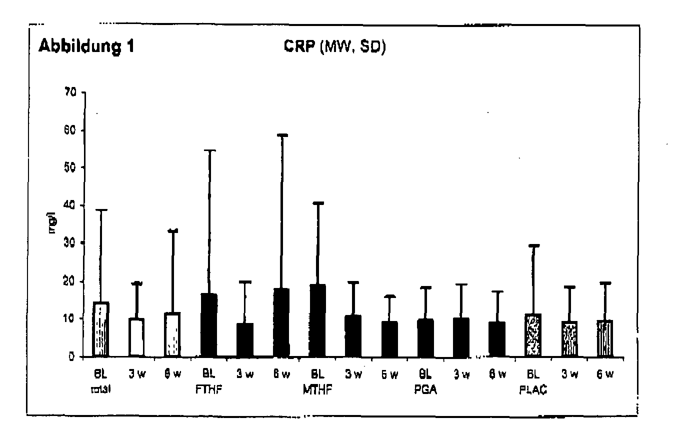

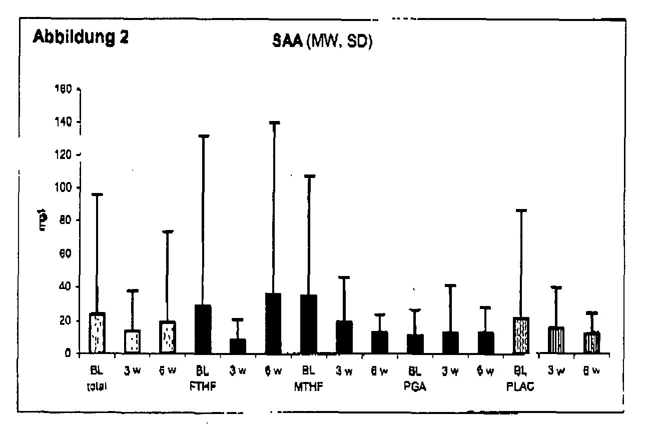

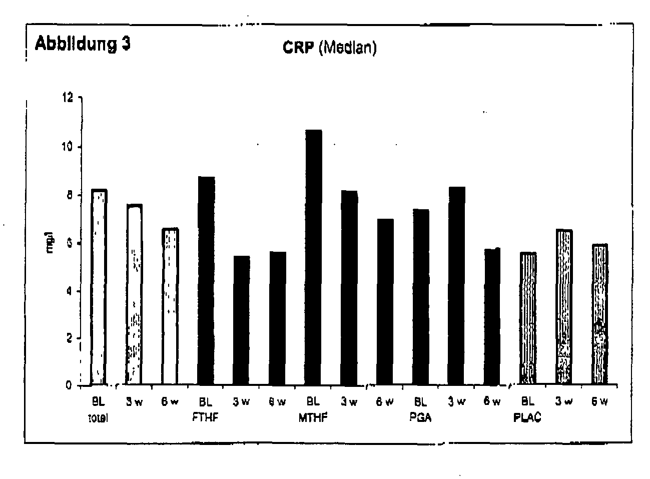

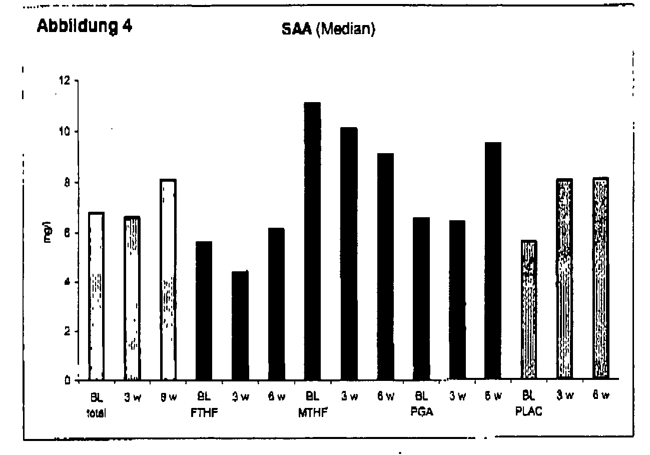

**Tabelle 3**

| | **total** | | | **FTHF** | | | **MTHF** | | | **PGA** | | | **PLAC** | | |
|---|---|---|---|---|---|---|---|---|---|---|---|---|---|---|---|
| | **BL** | **3w** | **6 w** | **BL** | **3w** | **6w** | **BL** | **3w** | **6 w** | **BL** | **3 w** | **6 w** | **BL** | **3w** | **6 w** |
| **CRP [n]** | 139 | 114 | 119 | 37 | 28 | 30 | 35 | 29 | 30 | 36 | 31 | 31 | 31 | 26 | 28 |
| **MW** | 14 | 10 | 11 | 16 | 9 | 18 | 19 | 11 | 9 | 10 | 10 | 9 | 11 | 9 | 10 |
| **SD** | 25 | 10 | 22 | 38 | 11 | 41 | 22 | 9 | 7 | 9 | 9 | 9 | 18 | 9 | 10 |
| **Median** | 8 | 8 | 7 | 9 | 5 | 6 | 11 | 6 | 7 | 7 | 8 | 6 | 6 | 7 | 6 |
| **SAA [n]** | 139 | 116 | 115 | 37 | 30 | 30 | 35 | 30 | 29 | 36 | 32 | 29 | 31 | 26 | 27 |
| **MW** | 24 | 14 | 19 | 28 | 6 | 35 | 34 | 19 | 13 | 11 | 12 | 13 | 21 | 15 | 13 |
| **8D** | 72 | 25 | 55 | 103 | 12 | 104 | 73 | 27 | 11 | 15 | 28 | 15 | 65 | 25 | 12 |
| **Median** | 7 | 7 | 8 | 6 | 4 | 6 | 11 | 10 | 9 | 7 | 6 | 10 | 6 | 8 | 9 |

Für die Patientlnnen, bei denen für jeden Zeitpunkt sämtliche Messwerte komplett erhoben werden konnten, wurde zusätzlich eine 'matched pair' Analyse durchgeführt. Dabei wurde jeweils für den einzelnen Patienten paarweise die Werte vor mit denen unter Therapie nach 3 und 6 Wochen verglichen. Tabelle 4 stellt die ermittelten Unterschiede für die inflammatorischen Marker und für Homocystein (HCYS) dar.

**Tabelle 4**

| | **Gesamt⁴** | | | **FTHF** | | | **MTHF** | | | **PGA** | | | **PLAC** | | |
|---|---|---|---|---|---|---|---|---|---|---|---|---|---|---|---|
| | 1 | 2 | 3 | 1 | 2 | 3 | 1 | 2 | 3 | 1 | 2 | 3 | 1 | 2 | 3 |
| Hcys | *** | *** | ∅ | * | ** | ∅ | ∅ | * | ∅ | * | * | ∅ | ∅ | ∅ | ∅ |
| CRP | ** | ** | Ø | * | ∅ | ∅ | * | ** | * | ∅ | ∅ | ∅ | ∅ | ∅ | ∅ |
| SAA | * | ∅ | ∅ | * | ∅ | * | ∅ | ∅ | ∅ | ∅ | ∅ | * | ∅ | ∅ | ∅ |

| | | | | | | | | | | | | | | | |
|---|---|---|---|---|---|---|---|---|---|---|---|---|---|---|---|
| ¹ BL vs 3 w, Vergleich zwischen Ausgangswert und 3 Wochenwert ² BL vs 6 w, Vergleich zwischen Ausgangswert und 6 Wochenwert ³ 3 w vs 6 w, Vergleich zwischen 3 und 6 Wochenwert ⁴ Gesamt, d.h. alle Daten bis auf die Werte der Placebogruppe * ie. p < 0.05, ** ie. p < 0.01, *** ie. p < 0.0001 | | | | | | | | | | | | | | | |

Wie auch in den Abbildungen 1 bis 4 dargestellt, zeigen die Entzündungsmarker CRP und SAA einen teilweise signifikanten Abfall unter der Folattherapie. Dabei scheinen die reduzierten Folate einen stärkeren Effekt zu erreichen. CRP reagiert am deutlichsten. Bei der Placebogruppe ändern sich CRP und SAA im Studienverlauf nicht.

### Schlussfolgerungen aus der klinischen Studie

Patientlnnen mit chronischer Niereninsuffizienz und Hämodialysebehandlung haben eine erhöhte inflammatorische Last, charakterisiert und in der vorliegenden Studie bestätigt durch deutlich pathologische CRP und SAA-Werte. Eine Korrelation mit den ebenfalls erhöhten Homocysteinwerten konnte nicht festgestellt werden und es scheint sich demnach um zwei unabhängige Prozesse zu handeln.
Die Therapie mit Folaten induziert im Trend, teilweise sogar statistisch signifikant, einen Abfall insbesondere der CRP-Spiegel. Die mit 5-Methyl-(6R,S)-tetahydrofolsäure behandelten Patientlnnen zeigen den stärksten Abfall bei den Entzündungsmarkern.

Der Einfluss von Folaten auf Akute-Phase Parameter ist umso überraschender, als bei der kleinen Zahl und sehr kurzen Studien- und damit Therapiedauer Trends und teilweise sogar signifikante Effekte beobachtet werden können.

Folate, insbesonders reduzierte Folgte, im Speziellen MTHF, senken, ähnlich den Statinen, die Akute-Phase Antwort, abzulesen an CRP und SAA und haben deshalb eine anti-inflammatorische Wirkung - akut und chronisch.

## Patentansprüche

1. Pharmazeutische Zusammensetzung zur Reduktion, Verlangsamung des Ansteigens oder sonstigen Beeinflussung von Entzündungen und entzündungsassoziierter Krankheiten durch Hemmung, Inhibierung oder sonstige Reduktion von CRP- oder SAA-Spiegeln beeinhaltend als aktive Wirksubstanz mindestens ein Folat oder ein pharmazeutisch verträgliches salz davon.

2. Pharmazeutische Zusammensetzung zur Reduktion, Verlangsamung des Ansteigens oder sonstigen Beeinflussung von Entzündungen und entzündungsassoziierter Krankheiten durch Hemmung, Inhibierung oder sonstige Reduktion von CRP- oder SAA-Spiegeln gemäss Anspruch 1
beeinhaltend als aktive Wirksubstanz ein Folat oder ein pharmazeutisch verträgliches Salz davon.

3. Pharmazeutische Zusammensetzung gemäss Anspruch 1 oder 2, worin das Folat oder pharmazeutisch verträglichen Salz davon ausgewählt ist aus der Gruppe bestehend aus Pteroinsäure-Monoglutamat (Folsäure), Dihydrofolsäure, 5-Formyltetrahydrofolsäure, 5-Methyltetrahydrofolsäure, 5,10-Methylentetrahydrofolsäure, 5,10-Metenyltetrahydrofolsäure, 10-Formyltetrahydrofolsäure oder Tetrahydrofolsäure, deren Polyglutamate, deren optische Isomeren, im speziellen deren optische reine natürliche Isomeren, Mischungen von optischen Isomeren, insbesondere racemische Mischungen, sowie auch deren pharmazeutisch verträgliche Salze.

4. Pharmazeutische Zusammensetzung gemäss Anspruch 1 oder 2, worin das Folat oder pharmazeutisch verträglichen Salz davon Folsäure, 5-Methyl-(6S)-tetrahydrofolsäure, 5-Methyl-(6R,S)-tetrahydrofolsäure, 5-Formyl-(6S)-tetrahydrofolsäure, 5-Formyl-(6R,S)-tetrahydrofolsäure ist.

5. Pharmazeutische Zusammensetzung gemäss einem der Ansprüche 1 bis 4 beinhaltend weitere Hilfsstoffe, wie Stabilisatoren, Additive, Antioxidantien, Puffer, Bakteriostatikas.

6. Pharmazeutische Zusammensetzung gemäss einem der Ansprüche 1 bis 5 beinhaltend weitere Zusatzstoffe, die im Folatzyklus eine Rolle spielen, wie Vitamine, Antioxidantien, Radikalfänger, Biopterine.

## Claims

1. Pharmaceutical preparation for the reduction, slowing down of the escalation or other influencing of inflammation and diseases associated with inflammation by blocking, Inhibition or other reduction of CRP and SAA levels, containing at least one folate or a pharmaceutically acceptable salt thereof as an active substance.

2. Pharmaceutical preparation for the reduction, slowing down of the escalation or other influencing of Inflammation and diseases associated with inflammation by blocking, Inhibition or other reduction of CRP and SAA levels according to claim 1, containing at least one folate or a pharmaceutically acceptable salt thereof as an active substance.

3. Pharmaceutical preparation according to claim 1 or 2, in which the folate or the pharmaceutically acceptable salt thereof is selected from the group consisting of pteroic acid monoglutamate (folic acid), dihydrofolic acid, 5-formyltetrahydrofolic acid, 5-methyltetrahydrofolic acid, 5,10-methylenetetrahydrofollc acid, 5,10-methenyltetrahydrofolic acid, 10-formyltetrahydrofolic acid or tetrahydrofolic acid, the polyglutamates thereof, the optical isomers thereof, especially the optical, purely natural isomers thereof, mixtures of optical isomers, especially racemic mixtures as well as the pharmaceutically acceptable salts thereof.

4. Pharmaceutical preparation according to claim 1 or 2, in which the folate or the pharmaceutically acceptable salt thereof Is folic acid, 5-methyl-(6S)- tetrahydrofolic acid, 5-methyl-(6R,S)- tetrahydrofolic acid, 5-formyl-(6S)-tetrahydrofolic acid, 5-formyl-(6R,S)-tetrahydrofolic acid.

5. Pharmaceutical preparation according to one of claims 1 to 4 containing further excipients, such as stabilisers, additives, antioxidants, buffers, bacteriostatics.

6. Pharmaceutical preparation according to one of claims 1 to 5 containing further additives, which play a role In the folate cycle, such as vitamins, antioxidants, radical scavengers, biopterins.

## Revendications

1. Composition pharmaceutique pour la réduction, le ralentissement de la croissance ou d'autres influences sur les inflammations et les maladies associées à des inflammations, par ralentissement, inhibition ou autre réduction des taux de CRP ou de SAA, contenant comme substance active au moins un folate ou un sel pharmaceutiquement acceptable de celui-ci.

2. Composition pharmaceutique pour la réduction, le ralentissement de la croissance ou d'autres influences sur les inflammations et les maladies associées à des inflammations, par ralentissement, inhibition ou autre réduction des taux de CRP ou de SAA selon la revendication 1, contenant comme substance active un folate ou un sel pharmaceutiquement acceptable de celui-ci.

3. Composition pharmaceutique selon la revendication 1 ou 2, dans laquelle le folate ou le sel pharmaceutiquement acceptable de celui-ci est choisi parmi le groupe constitué du monoglutamate d'acide ptéroique (acide folique), de l'acide dihydrofolique, de l'acide 5-formyl-tétrahydrofolique, de l'acide 5-méthyl-tétrahydrofolique, de l'acide 5,10-méthylène-tétrahydrofolique, de l'acide 5,10-méthényl-tétrahydrofolique, de l'acide 10-formyl-tétrahydrofolique ou de l'acide tétrahydrofolique, de leurs polyglutamates, de leurs isomères optiques, en particulier de leurs isomères optiques naturels purs, de mélanges d'isomères optiques, en particulier de mélanges racémiques, ainsi que, également, de leurs sels pharmaceutiquement acceptables.

4. Composition pharmaceutique selon la revendication 1 ou 2, dans laquelle le folate ou le sel pharmaceutiquement acceptable de celui-ci est l'acide folique, l'acide 5-méthyl-(6S)-tétrahydrofolique, l'acide 5-méthyl-(6R,S)-tétrahydrofolique, l'acide 5-formyl-(6S)-tétrahydrofolique, l'acide 5-formyl-(6R,s)-tétrahydrofolique.

5. Composition pharmaceutique selon l'une quelconque des revendications 1 à 4, contenant d'autres substances auxiliaires comme des stabilisateurs, des additifs, des antioxydants, des tampons, des bactériostatiques.

6. Composition pharmaceutique selon l'une quelconque des revendications 1 à 5, contenant d'autres additifs jouant un rôle dans le cycle du folate, comme des vitamines, des antioxydants, des capteurs de radicaux, de la bioptérine.
